# EUROPÄISCHE PATENTSCHRIFT

(11) **EP 3 413 950 B1**
(45) Veröffentlichungstag und Bekanntmachung des Hinweises auf die Patenterteilung: **17.03.2021**
(21) Anmeldenummer: 17700066.8
(22) Anmeldetag: 04.01.2017
(51) Int. Cl.: A61M 1/16

(54) **VORRICHTUNG ZUR EXTRAKORPORALEN BLUTBEHANDLUNG**
APPARATUS FOR EXTRACORPOREAL BLOOD TREATMENT
DISPOSITIF DE TRAITEMENT EXTRACORPOREL DU SANG

(30) Priorität: 10.02.2016 DE 102016001478
(43) Veröffentlichungstag der Anmeldung: 19.12.2018
(73) Patentinhaber: Fresenius Medical Care Deutschland GmbH, 61352 Bad Homburg v.d.H (DE)
(72) Erfinder: HÄCKER, Jürgen, 61267 Neu-Anspach (DE)
(74) Vertreter: Oppermann, Frank
(86) Internationale Anmeldenummer: PCT/EP2017/050155
(87) Internationale Veröffentlichungsnummer: WO 2017/137178

(56) Entgegenhaltungen:
- WO-A1-2012/052151
- DE-A1- 2 544 258
- US-A1- 2011 120 302
- US-A1- 2014 220 699

## Beschreibung

Die Erfindung betrifft eine Vorrichtung zur extrakorporalen Blutbehandlung, die über eine Einrichtung zur Bereitstellung von frischer Dialysierflüssigkeit und einen Ablauf für verbrauchte Dialysierflüssigkeit, eine Bilanziereinrichtung zur Bilanzierung von frischer Dialysierflüssigkeit und verbrauchter Dialysierflüssigkeit und einen Dialysator verfügt, der von einer semipermeablen Membran in eine Blutkammer und eine Dialysierflüssigkeitskammer unterteilt ist.

Die bekannten extrakorporalen Blutbehandlungsvorrichtungen weisen einen extrakorporalen Blutkreislauf auf, der die Blutkammer eines Dialysators einschließt, und ein Dialysierflüssigkeitssystem auf, das die Dialysierflüssigkeitskammer eines Dialysators einschließt. Blutkreislauf und Dialysierflüssigkeitssystem sind von der semipermeablen Membran des Dialysators getrennt. In dem Dialysierflüssigkeitssystem strömt frische Dialysierflüssigkeit von einer Einrichtung zur Bereitstellung von frischer Dialysierflüssigkeit in die Dialysierflüssigkeitskammer, während verbrauchte Dialysierflüssigkeit aus der Dialysierflüssigkeitskammer in den Ablauf strömt. Zur Bilanzierung von frischer und verbrauchter Dialysierflüssigkeit verfügen die bekannten Blutbehandlungsvorrichtungen über eine Bilanziereinrichtung. Darüber hinaus weisen die bekannten Blutbehandlungsvorrichtungen im Allgemeinen eine Ultrafiltrationseinrichtung auf, mit der dem Dialysierflüssigkeitssystem ein vorgegebenes Volumen an Dialysierflüssigkeit entzogen werden kann. Da die Bilanziereinrichtung sicherstellt, dass das Volumen der frischen Dialysierflüssigkeit gleich dem Volumen der verbrauchten Dialysierflüssigkeit ist, wird dem Patienten beim Betrieb der Ultrafiltrationseinrichtung über die semipermeable Membran des Dialysators Ultrafiltrat entzogen.

Der Teil des Dialysierflüssigkeitssystems, in dem frische Dialysierflüssigkeit über eine Dialysierflüssigkeits-Zuführleitung in die Dialysierflüssigkeitskammer des Dialysators strömt, wird nachfolgend als Primärseite und der Teil des Dialysierflüssigkeitssystems, in dem verbrauchte Dialysierflüssigkeit über eine Dialysierflüssigkeits-Abführleitung in den Ablauf strömt als Sekundärseite bezeichnet. Es sind Blutbehandlungsvorrichtungen bekannt, die sowohl auf der Primär- als auch Sekundärseite des Dialysierflüssigkeitssystems über eine Luftabscheidevorrichtung verfügen, die ein Behältnis mit einem Einlass für frische bzw. verbrauchte Dialysierflüssigkeit und einen Auslass für frische bzw. verbrauchte Dialysierflüssigkeit aufweisen. Einlass und Auslass sind derart angeordnet, dass sich in dem Behältnis ein Flüssigkeitsspiegel ausbildet, der ansteigen oder sinken kann. Oberhalb des Flüssigkeitsspiegels sammelt sich in dem Behältnis die abgeschiedene Luft. Diese Luftabscheidevorrichtungen stellen sicher, dass das Hydrauliksystem der Blutbehandlungsvorrichtung entlüftet wird.

Auf der Sekundärseite ist die Luftabscheideeinrichtung stromauf der Bilanziereinrichtung unmittelbar vor der Abzweigung angeordnet, an der Ultrafiltrat dem Dialysierflüssikeitssystem entzogen wird. Wenn Ultrafiltrat dem Dialysierflüssigkeitssystem entzogen wird, kann der Flüssigkeitsspiegel durch Ansammeln von Luft in dem Behältnis der Luftabscheideeinrichtung absinken. Wenn der Füllstand einen vorgegebenen Pegel unterschreitet, muss der Füllstand in dem Behältnis durch Abführen von Luft wieder angehoben werden.

Für eine korrekte Bilanzierung ist beim Betrieb der Blutbehandlungsvorrichtung zu beachten, dass die während der Blutbehandlung sich in dem Behältnis der Luftabscheideeinrichtung sammelnde Luft die Flüssigkeitsbilanz um das entsprechende Volumen verändern kann.

Aus der WO 2012/052151 A1 ist eine Blutbehandlungsvorrichtung bekannt, die über eine Einrichtung zur Bereitstellung von frischer Dialysierflüssigkeit und einem Ablauf für verbrauchte Dialysierflüssigkeit, einen Dialysator, eine Bilanziereinrichtung zur Bilanzierung von frischer Dialysierflüssigkeit und verbrauchter Dialysierflüssigkeit und eine Ultrafiltrationseinrichtung verfügt. In die Dialysierflüssigkeit-Abführleitung ist eine Luftabscheideeinrichtung geschaltet. Aus der DE2544258 ist eine Einrichtung für die Hämodialyse bekannt, welche eine Bilanziereinrichtung aufweist. Mittels einer Pumpeneinrichtung wird dem System Flüssigkeit entzogen oder Luft aus einem Luftausscheider abgesaugt.

Es sind Blutbehandlungsvorrichtungen bekannt, die zum Anheben des Füllstandes der Luftabscheideeinrichtung auf der Sekundärseite über eine zusätzliche Verbindungsleitung zu der Luftabscheideeinrichtung auf der Primärseite verfügen, so dass dem Behältnis der Luftabscheideeinrichtung auf der Sekundärseite zum Anheben des Flüssigkeitsspiegels frische Dialysierflüssigkeit zugeführt werden kann. Bei diesen Blutbehandlungsvorrichtungen erweist sich die Fluidverbindung zwischen Primär- und Sekundärseite aber als nachteilig, da diese zu einer Kontamination von frischer Dialysierflüssigkeit mit verbrauchter Dialysierflüssigkeit führen kann. Andere Blutbehandlungsvorrichtungen verfügen über eine zusätzliche Pumpe zum Zuführen von Dialysierflüssigkeit in das Behältnis der Luftabscheideeinrichtung, die in der zum Abfluss führenden Ablaufleitung angeordnet ist. Nachteilig ist, dass eine zusätzliche Komponente erforderlich ist, was auch einen erhöhten Wartungsaufwand zur Folge hat.

Der Erfindung liegt die Aufgabe zugrunde, eine Vorrichtung zur extrakorporalen Blutbehandlung bereitzustellen, die eine Entlüftung auf der Sekundärseite des Dialysierflüssigkeitssystems erlaubt. Der Erfindung liegt insbesondere die Aufgabe zugrunde, eine extrakorporale Blutbehandlungsvorrichtung zu schaffen, bei der die Primär- und Sekundärseite des Dialysierflüssigkeitssystems voneinander getrennt sind und die einen vereinfachten konstruktiven Aufbau hat. Darüber hinaus ist eine Aufgabe der Erfindung, die Entlüftung der Sekundärseite des Dialysierflüssigkeitssystems einer extrakorporalen Blutbehandlungsvorrichtung ohne den Einsatz zusätzlicher Komponenten zu ermöglichen, wobei nicht die Gefahr einer Kontamination von frischer Dialysierflüssigkeit mit verbrauchter Dialysierflüssigkeit besteht.

Die Lösung dieser Aufgaben erfolgt erfindungsgemäß mit den Merkmalen der unabhängigen Ansprüche. Die abhängigen Ansprüche betreffen vorteilhafte Ausführungsformen der Erfindung.

Die erfindungsgemäße Vorrichtung zur extrakorporalen Blutbehandlung verfügt über eine Ultrafiltrationseinrichtung zum Abziehen von verbrauchter Dialysierflüssigkeit aus dem Dialysierflüssigkeitssystem und Zuführen der verbrauchten Dialysierflüssigkeit zu dem Auslass unter Umgehung der Bilanziereinrichtung. Eine derartige Ultrafiltrationseinrichtung gehört zum Stand der Technik. Die Ultrafiltrationseinrichtung steht mit dem Behältnis einer Luftabscheideeinrichtung in Fluidverbindung, die auf der Sekundärseite des Dialysierflüssigkeitssystems angeordnet ist. Daher wird beim Betrieb der Ultrafiltrationseinrichtung Dialysierflüssigkeit aus dem Behältnis abgezogen, wodurch der Flüssigkeitsspiegel in dem Behältnis absinkt. Die Ultrafiltrationseinrichtung kann aus dem Behältnis der Luftabscheideeinrichtung direkt Flüssigkeit abziehen oder an eine zu dem Behältnis führende oder von dem Behältnis abgehende Leitung angeschlossen sein.

Die erfindungsgemäße Vorrichtung beruht auf der Verwendung der Ultrafiltrationseinrichtung der Blutbehandlungsvorrichtung zum Anheben des Flüssigkeitsspiegels in der Luftabscheideeinrichtung auf der Sekundärseite des Dialysierflüssigkeitssystems.

Bei der erfindungsgemäßen Vorrichtung ist die Ultrafiltrationseinrichtung derart ausgebildet ist, dass die Ultrafiltrationseinrichtung nicht nur in einem Ultrafiltrationsmodus, sondern auch in einem Entlüftungsmodus betrieben werden kann. Folglich erfüllt die Ultrafiltrationseinrichtung zwei unterschiedliche Aufgaben. In dem Ultrafiltrationsmodus wird die Ultrafiltrationseinrichtung derart betrieben, dass verbrauchte Dialysierflüssigkeit aus dem Behältnis der Luftabscheideeinrichtung abgezogen wird. Demgegenüber wird in dem Entlüftungsmodus die Ultrafiltrationseinrichtung in umgekehrter Richtung betrieben, so dass verbrauchte Dialysierflüssigkeit dem Behältnis der Luftabscheideeinrichtung zugeführt wird, wodurch der Flüssigkeitsspiegel in dem Behältnis der ersten Luftabscheideeinrichtung ansteigt. Mit dem Ansteigen des Flüssigkeitsspiegels wird die in dem Behältnis befindliche Luft verdrängt.

Der Verzicht auf zusätzliche Komponenten vereinfacht den Aufbau und verringert den Wartungsaufwand der Blutbehandlungsvorrichtung im Vergleich zu den bekannten Blutbehandlungsvorrichtungen. Mit der Reduzierung der Komponenten vereinfacht sich auch die Reinigung des Dialysierflüssigkeitssystems. Darüber hinaus bleiben Primär- und Sekundärseite des Dialysierflüssigkeitssystems voneinander getrennt, so dass nicht die Gefahr einer Kontamination von frischer Dialysierflüssigkeit mit verbrauchter Dialysierflüssigkeit aufgrund einer Verbindung von Primär- und die Sekundärseite des Dialysierflüssigkeitssystems besteht. Die fehlende Verbindung zwischen Primär- und Sekundärseite verhindert auch eine Beeinflussung der beiden Teile des Dialysierflüssigkeitssystems durch Druckschwankungen beim Anheben der Flüssigkeitsspiegel in den jeweiligen Luftabscheideeinrichtungen.

Ein weiterer Vorteil der erfindungsgemäße Vorrichtung liegt in der exakten Bilanzierung des Patienten während der extrakorporalen Blutbehandlung, da die Blutbehandlungsvorrichtung einen volumenkontrollierten Ersatz der in dem Behältnis der Luftabscheideeinrichtung befindlichen Luft durch Flüssigkeit ermöglicht, so dass aufgrund der Ansammlung von Luft in dem Behältnis entstehende Fehler in der Bilanzierung korrigiert werden.

Eine bevorzugte Ausführungsform der Erfindung sieht eine zweite Luftabscheideeinrichtung vor, die ein Behältnis aufweist, das mit der ersten Luftabscheideeinrichtung und der Ultrafiltrationseinrichtung derart in Fluidverbindung steht, dass die in dem Entlüftungsmodus beim Ansteigen des Flüssigkeitsspiegels aus der ersten Luftabscheideeinrichtung abgeführte Luft in dem Behältnis der zweiten Luftabscheideeinrichtung gesammelt wird, während die Ultrafiltrationseinrichtung in umgekehrter Richtung zum Zuführen von verbrauchter Dialysierflüssigkeit in das Behältnis der ersten Luftabscheideeinrichtung betrieben wird. Die zweite Luftabscheideeinrichtung stellt sicher, dass die Ultrafiltrationseinrichtung in dem Entlüftungsmodus nicht Luft ansaugen kann. Die Ultrafiltrationseinrichtung kann die verbrauchte Dialysierflüssigkeit aus dem Behältnis der zweiten Luftabscheideeinrichtung abziehen, solange das Behältnis der zweiten Luftabscheideeinrichtung mit Dialysierflüssigkeit gefüllt ist, d. h. der Flüssigkeitsspiegel in dem Behältnis der zweiten Luftabscheideeinrichtung nicht unter einen vorgegebenen Wert abgesunken ist.

Eine weitere bevorzugte Ausführungsform sieht vor, dass die Blutbehandlungsvorrichtung eine Steuereinheit aufweist, die derart konfiguriert ist, dass die Ultrafiltrationseinrichtung wechselweise in dem Ultrafiltrationsmodus und in dem Entlüftungsmodus betrieben wird. Die bevorzugte Ausführungsform kann auch einen Betrieb vorsehen, bei dem nur einmalig aus dem Ultrafiltrationsmodus in den Belüftungsmodus gewechselt wird und danach die Behandlung im Ultrafiltrationsmodus fortgesetzt wird.

Die Steuereinheit kann eine separate Steuereinheit oder Bestandteil der zentralen Steuereinheit der Blutbehandlungsvorrichtung sein. Während des Absaugens von Flüssigkeit aus dem Behältnis der zweiten Luftabscheideeinrichtung durch die Ultrafiltrationseinrichtung in dem Entlüftungsmodus sinkt der Flüssigkeitspegel in dem Behältnis der zweiten Luftabscheideeinrichtung. Durch den Betrieb der Ultrafiltrationseinrichtung in dem Ultrafiltrationsmodus wird der Flüssigkeitspegel in dem Behältnis der zweiten Luftabscheideeinrichtung wieder angehoben. Der wechselweise Betrieb der Ultrafiltrationseinrichtung in dem Ultrafiltrations- und Entlüftungsmodus erlaubt das Fördern kleinerer Volumina an Flüssigkeit, so dass sichergestellt ist, dass das Behältnis der ersten Luftabscheideeinrichtung immer ausreichend mit Flüssigkeit gefüllt ist.

Die Steuereinheit ist bei einer weiteren bevorzugten Ausführungsform derart konfiguriert, dass die Ultrafiltrationseinrichtung in dem Ultrafiltrationsmodus für ein vorgegebenes erstes Zeitintervall und in dem Entlüftungsmodus für ein vorgegebenes zweites Zeitintervall betrieben wird. Die Zeitintervalle können derart bemessen sein, dass jeweils ein vorgegebenes Volumen an Flüssigkeit gefördert wird.

Zur Überwachung des Flüssigkeitsspiegels weist die erste Luftabscheideeinrichtung vorzugsweise einen Füllstandgeber auf, wobei die Steuereinheit derart konfiguriert ist, dass die Ultrafiltrationseinrichtung in dem Ultrafiltrationsmodus und dem Entlüftungsmodus solange wechselweise betrieben wird, bis der Füllstandgeber einen vorgegebenen Füllstand misst.

Bei einer besonders bevorzugten Ausführungsform weist das Behältnis der ersten Luftabscheideeinrichtung einen Entlüftungsanschluss zum Abführen von Luft und einen Ultrafiltrationsanschluss zum Zuführen oder Abführen von verbrauchter Dialysierflüssigkeit auf, wobei der Entlüftungsanschluss oberhalb des Ultrafiltrationsanschlusses angeordnet ist, so dass verbrauchte Dialysierflüssigeit dem Behältnis unterhalb des Flüssigkeitsspiegels zugeführt oder aus dem Behältnis abgezogen werden kann. Das Behältnis der zweiten Luftabscheideeinrichtung weist vorzugsweise einen Einlass und einen Auslass auf, wobei der Einlass unterhalb des Auslasses angeordnet ist, und weist vorzugsweise einen Ultrafiltrationsanschluss zum Zuführen oder Abführen von verbrauchter Dialysierflüssigkeit auf, der unterhalb des Auslasses angeordnet ist, so dass verbrauchte Dialysierflüssigeit dem Behältnis unterhalb des Flüssigkeitsspiegels zugeführt oder aus dem Behältnis abgezogen werden kann.

Die Ultrafiltrationseinrichtung weist vorzugsweise eine Ultrafiltrationspumpe mit einem ersten und einem zweiten Anschluss aufweist, wobei der erste Anschluss über einen ersten Abschnitt einer Ultrafiltrationsleitung an den Ultrafiltrationsanschluss des Behältnisses der ersten Luftabscheideeinrichtung angeschlossen ist und der zweite Anschluss über einen zweiten Abschnitt der Ultrafiltrationsleitung an den Ultrafiltrationsanschluss des Behältnisses der zweiten Luftabscheideeinrichtung angeschlossen ist. Die Ultrafiltrationspumpe kann unterschiedlich ausgebildet sein, beispielsweise eine okkludierende Rollenpumpe sein, in die eine Schlauchleitung eingelegt werden kann. Die Ultrafiltrationspumpe kann auch eine Membranpumpe sein, die am Ein- und Auslass zusätzlich ein Einlass- bzw. Auslassventil aufweist. Zum Messen der ultrafiltrierten Flüssigkeitsmenge kann die Ultrafiltrationseinrichtung über eine geeignete Messeinrichtung verfügen. Bei einer Rollenpumpe können beispielsweise die Umdrehungen und bei einer Membranpumpe die Pumpenhübe gezählt werden, um die Flüssigkeitsmenge zu bestimmen. Alternativ kann auch der Volumenstrom mit einem Flusssensor und einer Auswerteinheit erfasst werden.

Der Entlüftungsanschluss des Behältnisses der ersten Luftabscheideeinrichtung ist über eine Entlüftungsleitung an den Einlass des Behältnisses der zweiten Luftabscheideeinrichtung angeschlossen und der Auslass des Behältnisses der zweiten Luftabscheideeinrichtung ist über einen dritten Abschnitt der Ultrafiltrationsleitung mit dem Ablauf verbunden, wobei in der Entlüftungsleitung ein erstes Absperrorgan und in dem dritten Abschnitt der Ultrafiltrationsleitung ein zweites Absperrorgan angeordnet ist. Mit den Absperrorganen können die Fluidverbindungen zwischen den Behältnissen hergestellt oder unterbrochen werden.

Die Steuereinheit ist vorzugsweise derart konfiguriert, dass in dem Ultrafiltrationsmodus das erste Absperrorgan geschlossen und das zweite Absperrorgan geöffnet ist. Dadurch ist sichergestellt, dass verbrauchte Dialysierflüssigkeit aus dem Behältnis der ersten Ultrafiltrationseinrichtung abgezogen und unter Umgehung der Bilanziereinrichtung in den Ablauf gefördert werden kann. Weiterhin ist Steuereinheit derart konfiguriert, dass in dem Entlüftungsmodus das erste Absperrorgan geöffnet und das zweite Absperrorgan geschlossen ist, so dass verbrauchte Dialysierflüssigkeit aus dem Behältnis der zweiten Luftabscheideeinrichtung zum Anheben des Flüssigkeitsspiegels in das Behältnis der ersten Luftabscheideeinrichtung gepumpt werden kann.

Im Folgenden wird ein Ausführungsbeispiel der Erfindung unter Bezugnahme auf die Figur im Einzelnen beschrieben, die das Dialysierflüssigkeitssystem der extrakorporalen Blutbehandlungsvorrichtung in stark vereinfachter schematischer Darstellung zeigt, wobei die Ultrafiltrationseinrichtung in einem Ultrafiltrationsmodus und einem Entlüftungsmodus betrieben wird.

Die Vorrichtung zur extrakorporalen Blutbehandlung, insbesondere Dialysevorrichtung, weist eine Einrichtung 1 zur Bereitstellung von frischer Dialysierflüssigkeit und einen Ablauf 2 für verbrauchte Dialysierflüssigkeit auf. Unter einem Ablauf 2 wird beispielsweise ein Behälter zum Sammeln verbrauchter Dialysierflüssigkeit oder ein Abfluss verstanden. Darüber hinaus weist die extrakorporale Blutbehandlungsvorrichtung einen Dialysator 3 und eine Bilanziereinrichtung 4 auf. Der Dialysator 3 ist durch eine semipermeable Membran 5 in eine Blutkammer 6 und eine Dialysierflüssigkeitskammer 7 unterteilt. Zu dem Einlass der Blutkammer 6 führt eine arterielle Blutleitung 8 und von dem Auslass der Blutkammer 6 geht eine venöse Blutleitung 9 ab. Die arterielle und venöse Blutleitung 8, 9 und die Blutkammer 6 des Dialysators 3 bilden den extrakorporalen Blutkreislauf I der Blutbehandlungsvorrichtung. Die semipermeable Membran 5 des Dialysators 3 trennt den extrakorporalen Blutkreislauf I von dem Dialysierflüssigkeitssystem II der Blutbehandlungsvorrichtung.

Von der Einrichtung 1 zur Bereitstellung von frischer Dialysierflüssigkeit führt eine Dialysierflüssigkeit-Zuführleitung 10 zu dem Einlass der Dialysierflüssigkeitskammer 7 und von dem Auslass der Dialysierflüssigkeitskammer 7 führt eine Dialysierflüssigkeit-Abführleitung 11 zu dem Ablauf 2, so dass frische Dialysierflüssigkeit von der Einrichtung 1 zur Bereitstellung von frischer Dialysierflüssigkeit durch die Dialysierflüssigkeitskammer 7 in den Ablauf 2 strömen kann.

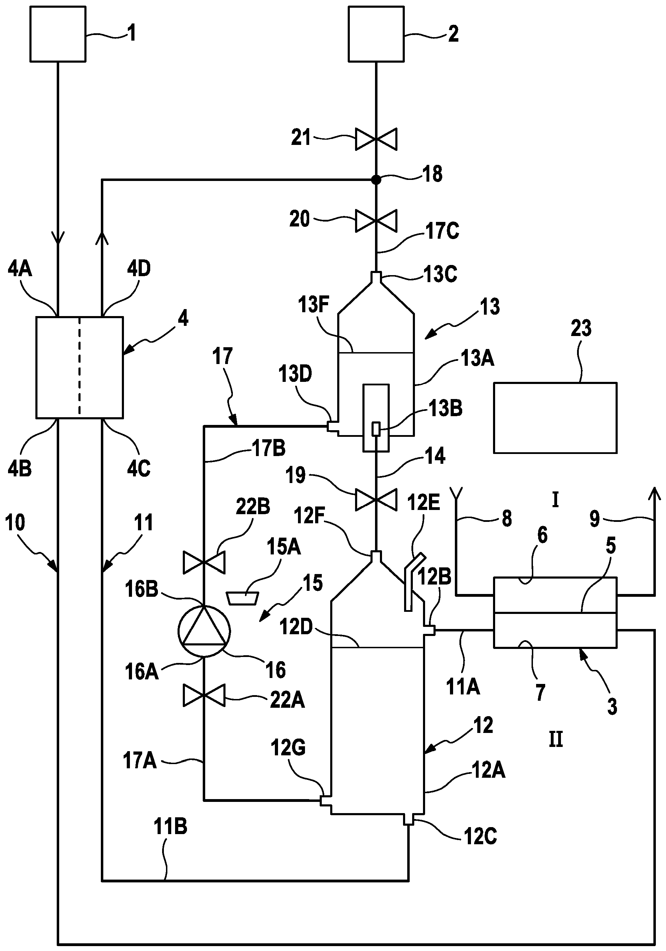

Die Bilanziereinrichtung 4 bilanziert frische gegen verbrauchte Dialysierflüssigkeit. Dadurch wird sichergestellt, dass das Volumen an frischer Dialysierflüssigkeit dem Volumen an verbrauchter Dialysierflüssigkeit entspricht. Die in Fig. 1 nur schematisch dargestellte Bilanziereinrichtung 4, die einen primärseitigen Einlass 4A und Auslass 4B für frische Dialysierflüssigkeit und einen sekundärseitigen Einlass 4C und Auslass 4D für verbrauchte Dialysierflüssigkeit aufweist, ist in die Dialysierflüssigkeit-Zuführleitung 10 und die Dialysierflüssigkeit-Abführleitung 11 geschaltet, so dass frische Dialysierflüssigkeit über die Primärseite und verbrauchte Dialysierflüssigkeit über die Sekundärseite der Bilanziereinrichtung 4 strömt. Zur Bilanzierung von frischer und verbrauchter Dialysierflüssigkeit kann die Bilanziereinrichtung über Bilanzkammern verfügen. Der Volumenstrom kann zur Bilanzierung aber auch mit Flusssensoren überwacht werden. Bilanziereinrichtungen unterschiedlicher Ausbildung gehören zum Stand der Technik.

Das Dialysierflüssigkeitssystem II weist eine Primärseite und eine Sekundärseite auf. Die Primärseite umfasst den Teil des Dialysierflüssigkeitssystems, der stromauf der Dialysierflüssigkeitskammer liegt, während die Sekundärseite den Teil des Dialysierflüssigkeitssystems II umfasst, der stromab der Dialysierflüssigkeitskammer 7 des Dialysators 3 liegt. In der Dialysierflüssigkeit befindliche Luft (Luftblasen) wird sowohl auf der Primär- als auch Sekundärseite des Dialysierflüssigkeitssystems II abgeschieden.

In Fig. 1 sind nur diejenigen Komponenten des Luftabscheidesystems dargestellt, die sich auf der Sekundärseite des Dialysierflüssigkeitssystems II befinden. Das Luftabscheidesystem weist eine erste Luftabscheideeinrichtung 12 und eine zweite Luftabscheideeinrichtung 13 auf.

Die erste Luftabscheideeinrichtung 12 weist ein Behältnis 12A mit einem Einlass 12B und einem Auslass 12C auf. Der Einlass 12B befindet sich oberhalb des Auslasses 12C, der im Bereich des Bodens des Behältnisses 12A angeordnet ist. Der Auslass der Dialysierflüssigkeitskammer 7 des Dialysators 3 ist über einen ersten Leitungsabschnitt 11A der Dialysierflüssigkeit-Abführleitung 11 mit dem Einlass 12B der ersten Luftabscheideeinrichtung 12 verbunden, während der Auslass 12C der ersten Luftabscheideeinrichtung 12 über einen zweiten Leitungsabschnitt 11B mit dem Ablauf 2 verbunden ist. Die erste Luftabscheideeinrichtung 12 kann beispielsweise eine konventionelle Tropfkammer sein, die zum Stand der Technik gehört.

Während des Betriebs der Blutbehandlungsvorrichtung strömt verbrauchte Dialysierflüssigkeit durch das Behältnis 12A der ersten Luftabscheideeinrichtung 12, so dass sich in dem Behältnis 12A ein Flüssigkeitsspiegel 12D ausbildet. Zur Detektion des Flüssigkeitspegels in dem Behältnis 12A weist die erste Luftabscheideeinrichtung 12 einen Füllstandgeber 12E auf.

Die mit der ersten Luftabscheideeinrichtung 12 abgeschiedene Luft (Luftblasen) sammeln sich in dem Raum des Behältnisses 12A oberhalb des Flüssigkeitsspiegels 12D.

Die zweite Luftabscheideeinrichtung 13, die ebenfalls eine konventionelle Tropfkammer sein kann, weist ein Behältnis 13A mit einem Einlass 13B und einem Auslass 13C auf. Der Auslass 13C ist an dem Behältnis 13A oberhalb des Einlasses 13B angeordnet. In dem Behältnis 13A bildet sich während des Betriebs der Blutbehandlungsvorrichtung ein Flüssigkeitsspiegel 13F aus.

Das Behältnis 12A der ersten Luftabscheideeinrichtung 12 weist einen oberhalb des Ein- und Auslasses 12B, 12C angeordneten Entlüftungsanschluss 12F auf, der über eine Entlüftungsleitung 14 mit dem Einlass 13B der zweiten Luftabscheideeinrichtung 13 verbunden ist.

Darüber hinaus verfügt die Blutbehandlungsvorrichtung über eine Ultrafiltrationseinrichtung 15, mit der dem Patienten eine vorgegebene Menge an Ultrafiltrat über die semipermeable Membran 5 des Dialysators 3 entzogen werden kann. Die Ultrafiltrationseinrichtung 15 weist eine Ultrafiltrationspumpe 16 mit einem ersten Anschluss 16A und einem zweiten Anschluss 16B auf. Der erste Anschluss 16A der Ultrafiltrationspumpe 16 ist über einen ersten Abschnitt 17A der Ultrafiltrationsleitung 17 mit einem Ultrafiltrationsanschluss 12G verbunden, der an dem Behältnis 12A der ersten Luftabscheideeinrichtung 12 unterhalb des Einlasses 12B und des Entlüftungsanschlusses 12F vorzugsweise im Bereich des Bodens des Behältnisses 12A angeordnet ist. Der zweite Anschluss 16B der Ultrafiltrationspumpe 16 ist über einen zweiten Abschnitt 17B der Ultrafiltrationsleitung 12 mit einem Ultrafiltrationsanschluss 13D verbunden, der an dem Behältnis 13A der zweiten Luftabscheideeinrichtung 13 unterhalb des Auslasses 13B vorzugsweise im Bereich des Bodens des Behältnisses vorgesehen ist. Von dem Auslass 13C des Behältnisses 13A der zweiten Luftabscheideeinrichtung 13 führt ein dritter Abschnitt der Ultrafiltrationsleitung 17 zu dem Abfluss 2. Der dritte Abschnitt 17B der Ultrafiltrationsleitung ist hierzu an einem Anschlusspunkt 18 an dem zweiten Leitungsabschnitt 11B der Dialysierflüssigkeit-Abführleitung 11 angeschlossen, der stromab der Bilanziereinrichtung 14 liegt.

Die Ultrafiltrationspumpe 16 kann eine okkludierende Rollenpumpe oder eine Membranpumpe sein. Wenn die Ultrafiltrationspumpe 16 eine Membranpumpe ist, sind in Ultrafiltrationsleitung 17 stromauf der Pumpe ein Einlassventil 22A und stromab der Pumpe ein Auslassventil 22B angeordnet. Ein- und Auslassventil 22A, 22B sind nicht erforderlich, wenn die Ultrafiltrationspumpe eine okkludierende Pumpe ist.

Zum Unterbrechen der Fluidverbindung ist an der Entlüftungsleitung 14 ein erstes Absperrorgan 19, an dem dritten Abschnitt 17C der Ultrafiltrationsleitung 17 ein zweites Absperrorgan 20 und an dem Abschnitt des zweiten Leitungsabschnitts 11B der Dialysierflüssigkeit-Abführleitung 11 stromab des Anschlusspunktes 18 ein drittes Absperrorgan 21 vorgesehen.

Die Ultrafiltrationseinrichtung 15 verfügt weiterhin über eine nur andeutungsweise dargestellte Messeinrichtung 15A zum Messen der ultrafiltrierten Flüssigkeitsmenge. Die Messeinrichtung 15A zählt bei einer Rollenpumpe die Umdrehungen und bei einer Membranpumpe die Membranhübe, um die ultrafiltrierte Flüssigkeitsmenge zu bestimmen. Alternativ kann auch ein Flusssensor mit einer Auswerteinheit zur Messung der ultrafiltrierten Flüssigkeitsmenge vorgesehen sein.

Die Blutbehandlungsvorrichtung verfügt über eine zentrale Steuereinheit 23, mit der die einzelnen Komponenten der Blutbehandlungsvorrichtung gesteuert werden. Die zentrale Steuereinheit 23 kann eine Datenverarbeitungseinheit aufweisen, auf der ein Computerprogramm läuft. Die Steuereinheit 23 steuert über nicht dargestellte Steuerleitungen die Ultrafiltrationspumpe 16 der Ultrafiltrationseinrichtung 15 sowie die einzelnen Absperrorgane 19 bis 21 und das Ein- und Auslassventil 22A und 22B an.

Nachfolgend wird im Einzelnen beschrieben, wie die Steuereinheit die Ultrafiltrationspumpe und die Absperrorgane ansteuert, um Luft (Luftblasen) in der verbrauchten Dialysierflüssigkeit auf der Sekundärseite des Dialysierflüssigkeitssystems II abzuscheiden. Hierzu steuert die Steuereinheit 23 die Ultrafiltrationspumpe und die Absperrorgane derart an, dass die nachfolgenden Verfahrensschritte ausgeführt werden.

Zunächst wird die Ultrafiltrationseinrichtung 15 in einem Ultrafiltrationsmodus betrieben. Dieser entspricht dem Normalbetrieb der Dialysevorrichtung, wenn eine Ultrafiltration erfolgen soll. In dem Ultrafiltrationsmodus, der in Fig. 1 dargestellt ist, sind das zweite Absperrorgan 20 und das dritte Absperrorgan 21 geöffnet und das erste Absperrorgan 19 ist geschlossen. Ein- und Auslassventil 22A und 22B sind, sofern sie überhaupt vorhanden sind, ebenfalls geöffnet. Die Ultrafiltrationspumpe 16 entzieht in dem Ultrafiltrationsmodus dem Dialysierflüssigkeitssystem II aus dem Behältnis 12A der ersten Luftabscheideeinrichtung 12 verbrauchte Dialysierflüssigkeit, die unter Umgehung der Bilanziereinrichtung 4 in den Ablauf 2 strömt. Die verbrauchte Dialysierflüssigkeit strömt durch den ersten, zweiten und dritten Abschnitt 17A bis 17C der Ultrafiltrationsleitung 17 zu dem Verbindungsanschluss 18, so dass die Dialysierflüssigkeit in den Ablauf 2 fließen kann.

In der Dialysierflüssigkeit befindliche Luft (Luftblasen) wird in der ersten Luftabscheideeinrichtung 12 abgeschieden, die sich oberhalb des Flüssigkeitsspiegels 12D in dem Behältnis 12A der ersten Luftabscheideeinrichtung 12 sammelt. Während des Betriebs der Ultrafiltrationspumpe 16 kann der Flüssigkeitsspiegel 12D in der ersten Luftabscheideeinrichtung 12 absinken. Daher ist es erforderlich, den Flüssigkeitspegel wieder anzuheben.

Zum Anheben des Flüssigkeitspegels in der ersten Luftabscheideeinrichtung 12 steuert die Steuereinheit 23 die Ultrafiltrationseinrichtung 15 derart an, dass die Ultrafiltrationspumpe 16 in einem Entlüftungsmodus betrieben wird, in dem die Ultrafiltrationspumpe in entgegengesetzter Richtung läuft, d. h. die Förderrichtung der Pumpe umgekehrt ist. In dem Entlüftungsmodus öffnet die Steuereinheit 23 das erste Absperrorgan 19 und schließt das zweite Absperrorgan 20, so dass die Fluidverbindung zu dem Ablauf 2 unterbrochen ist. Die Ultrafiltrationspumpe 16 fördert nunmehr verbrauchte Dialysierflüssigkeit, die sich in dem Behältnis 13A der zweiten Luftabscheideeinrichtung 13 befindet, in das Behältnis 12A der ersten Luftabscheideeinrichtung 12, so dass der Flüssigkeitsspiegel 12D in der ersten Luftabscheideeinrichtung 12 ansteigt. Die Ultrafiltrationspumpe wird für ein vorgegebenes Zeitintervall betrieben, das derart bemessen ist, dass nur ein Teil der in der zweiten Luftabscheideeinrichtung 13 befindlichen Flüssigkeit in die erste Luftabscheideeinrichtung 12 gefördert wird. Daraufhin schaltet die Steuereinheit 23 die Ultrafiltrationseinrichtung 15 wieder in den Ultrafiltrationsmodus, wobei die Steuereinheit 23 wieder das erste Absperrorgan 19 schließt und das zweite Absperrorgan 20 öffnet. Während des Betriebs der Ultrafiltrationspumpe im Ultrafiltrationsmodus wird das Behältnis 13A der zweiten Luftabscheideeinrichtung 13 wieder mit verbrauchter Dialysierflüssigkeit befüllt. Der Betrieb im Ultrafiltrationsmodus erfolgt für ein vorgegebenes zweites Zeitintervall. Die Steuereinheit steuert die Ultrafiltrationseinrichtung 16 derart an, dass ein wechselweiser Betrieb der Ultrafiltrationseinrichtung im Ultrafiltrations- und Entlüftungsmodus solange erfolgt, bis der Füllstandgeber 12E der ersten Luftabscheideeinrichtung 12 einen vorgegebenen Füllstand detektiert, d. h. der Flüssigkeitsspiegel in der ersten Luftabscheideeinrichtung wieder auf den vorgegebenen Pegel angestiegen ist. Es ist auch möglich, dass ein nur einmaliger Wechsel vom Ultrafiltrationsmodus in den Entlüftungsmodus erfolgt, wonach die Behandlung dann im Ultrafiltrationsmodus fortgesetzt wird.

In dem Entlüftungsmodus wird die in dem Behältnis 12A der ersten Luftabscheideeinrichtung 12 befindliche Luft über die Entlüftungsleitung 14 in die zweite Luftabscheideeinrichtung 13 geleitet. Da diese Luft in der zweiten Luftabscheideeinrichtung abgeschieden wird, kann die Ultrafiltrationspumpe 16 in dem Entlüftungsmodus nicht Luft ansaugen, die zwischen den beiden Entlüftungseinrichtungen zirkulieren könnte.

Bei der erfindungsgemäßen Blutbehandlungsvorrichtung kann die aus dem Dialysierflüssigkeitssystem abgeschiedene Luft (Luftblasen) nicht zu Fehlbilanzierungen führen. Das Ersetzen der angesammelten Luft durch Flüssigkeit führt zur Korrektur des während der Dialyse aufgrund der Lufteinschlüsse im volumenkontrollierten bilanzierenden Kreislauf temporär auftretenden Bilanzfehlers.

### Bezugszeichenliste:

Einrichtung zur Bereitstellung von frischer Dialysierflüssigkeit 1
Ablauf 2
Dialysator 3
Bilanziereinrichtung 4
primärseitiger Einlass der Bilanziereinrichtung 4A,
primärseitiger Auslass der Bilanziereinrichtung 4B,
sekundärseitiger Einlass der Bilanziereinrichtung 4C
sekundärseitiger Auslass der Bilanziereinrichtung 4D
semipermeable Membran 5
Blutkammer 6
Dialysierflüssigkeitskammer 7
arterielle Blutleitung 8
venöse Blutleitung 9
extrakorporaler Blutkreislauf I
Dialysierflüssigkeitssystem II
Dialysierflüssigkeit-Zuführleitung 10
Dialysierflüssigkeit-Abführleitung 11
erster Leitungsabschnitt 11A der Dialysierflüssigkeit-Abführleitung
zweiter Leitungsabschnitt 11B der Dialysierflüssigkeit-Abführleitung
erste Luftabscheideeinrichtung 12
Behältnis 12A der ersten Luftabscheideeinrichtung
Einlass 12B der ersten Luftabscheideeinrichtung
Auslass 12C der ersten Luftabscheideeinrichtung
Flüssigkeitsspiegel 12D der ersten Luftabscheideeinrichtung
Füllstandgeber 12E
Entlüftungsanschluss 12F der ersten Luftabscheideeinrichtung
Ultrafiltrationsanschluss 12G der ersten Luftabscheideeinrichtung
zweite Luftabscheideeinrichtung 13
Behältnis 13A der zweiten Luftabscheideeinrichtung 13A
Einlass 13B der zweiten Luftabscheideeinrichtung
Auslass 13C der zweiten Luftabscheideeinrichtung
Ultrafiltrationsanschluss 13D der zweiten Luftabscheideeinrichtung
Flüssigkeitsspiegel 13F der zweiten Luftabscheideeinrichtung
Entlüftungsleitung 14
Ultrafiltrationseinrichtung 15
Messeinrichtung 15A zum Messen der ultrafiltrierten Flüssigkeitsmenge
Ultrafiltrationspumpe 16
erster Anschluss 16A der Ultrafiltrationspumpe
zweiter Anschluss 16B der Ultrafiltrationspumpe
Ultrafiltrationsleitung 17
erster Abschnitt 17A der Ultrafiltrationsleitung
zweiter Abschnitt 17B der Ultrafiltrationsleitung
dritter Abschnitt 17C der Ultrafiltrationsleitung
Anschlusspunkt 18
erstes Absperrorgan 19
zweites Absperrorgan 20
drittes Absperrorgan 21
Einlass- und Auslassventil 22A, 22B
Steuereinheit 23

## Patentansprüche

1. Vorrichtung zur extrakorporalen Blutbehandlung mit
einer Einrichtung (1) zur Bereitstellung von frischer Dialysierflüssigkeit und einem Ablauf (2) für verbrauchte Dialysierflüssigkeit,
einer Bilanziereinrichtung (4) zur Bilanzierung von frischer Dialysierflüssigkeit und verbrauchter Dialysierflüssigkeit,
einem Dialysator (3), der von einer semipermeablen Membran (5) in eine Blutkammer (6) und eine Dialysierflüssigkeitskammer (7) unterteilt ist,
einer von der Einrichtung (1) zur Bereitstellung von frischer Dialysierflüssigkeit über die Bilanziereinrichtung (4) zu einem Einlass der Dialysierflüssigkeitskammer (7) führenden Dialysierflüssigkeit-Zuführleitung (10) für frische Dialysierflüssigkeit und einer von einem Auslass der Dialysierflüssigkeitskammer (7) über die Bilanziereinrichtung (4) zu dem Ablauf (2) führenden Dialysierflüssigkeit-Abführleitung (11) für verbrauchte Dialysierflüssigkeit,
einer ersten Luftabscheideeinrichtung (12), die ein Behältnis (12A) mit einem Einlass (12B) zum Zuführen von verbrauchter Dialysierflüssigkeit und einem Auslass (12C) zum Abführen von verbrauchter Dialysierflüssigkeit aufweist, die derart angeordnet sind, dass sich in dem Behältnis (12A) ein Flüssigkeitsspiegel (12D) ausbildet, wobei die Dialysierflüssigkeit-Abführleitung (11) einen ersten Leitungsabschnitt (11A) aufweist, der von dem Auslass der Dialysierflüssigkeitskammer (7) zu dem Einlass (12A) der ersten Luftabscheideeinrichtung (12) führt, und einen zweiten Leitungsabschnitt (11B) aufweist, der von dem Auslass der ersten Luftabscheideeinrichtung (12) über die Bilanziereinrichtung (4) zu dem Ablauf (2) führt,
einer Ultrafiltrationseinrichtung (15) zum Abziehen von verbrauchter Dialysierflüssigkeit aus dem Behältnis (12A) der ersten Luftabscheideeinrichtung (12) und Zuführen der verbrauchten Dialysierflüssigkeit zu dem Ablauf (2) unter Umgehung der Bilanziereinrichtung (4), wobei die Ultrafiltrationseinrichtung (15) derart ausgebildet ist, dass
in einem Ultrafiltrationsmodus die Ultrafiltrationseinrichtung (15) zum Abziehen von verbrauchter Dialysierflüssigkeit aus dem Behältnis (12A) der ersten Luftabscheideeinrichtung (12) betrieben wird, **dadurch gekennzeichnet, dass** in einem Entlüftungsmodus die Ultrafiltrationseinrichtung (15) in umgekehrter Richtung zum Zuführen von verbrauchter Dialysierflüssigkeit in das Behältnis (12A) der ersten Luftabscheideeinrichtung (12) betrieben wird, so dass der Flüssigkeitsspiegel (12D) in dem Behältnis der ersten Luftabscheideeinrichtung ansteigt.

2. Vorrichtung zur extrakorporalen Blutbehandlung nach Anspruch 1, **dadurch gekennzeichnet, dass** eine zweite Luftabscheideeinrichtung (13) vorgesehen ist, die ein Behältnis (13A) aufweist, das mit der ersten Luftabscheideeinrichtung (12) und der Ultrafiltrationseinrichtung (15) derart in Fluidverbindung steht, dass die in dem Entlüftungsmodus beim Ansteigen des Flüssigkeitsspiegels (12D) aus der ersten Luftabscheideeinrichtung (12) abgeführte Luft in dem Behältnis (13A) der zweiten Luftabscheideeinrichtung (13) gesammelt wird, während die Ultrafiltrationseinrichtung (15) in umgekehrter Richtung zum Zuführen von verbrauchter Dialysierflüssigkeit in das Behältnis (12A) der ersten Luftabscheideeinrichtung (12) betrieben wird.

3. Vorrichtung zur extrakorporalen Blutbehandlung nach Anspruch 1 oder 2, **dadurch gekennzeichnet, dass** die Blutbehandlungsvorrichtung eine Steuereinheit (23) aufweist, die derart konfiguriert ist, dass die Ultrafiltrationseinrichtung (15) wechselweise in dem Ultrafiltrationsmodus und in dem Entlüftungsmodus betrieben wird.

4. Vorrichtung zur extrakorporalen Blutbehandlung nach Anspruch 3, **dadurch gekennzeichnet, dass** die Steuereinheit (23) derart konfiguriert ist, dass die Ultrafiltrationseinrichtung (15) in dem Ultrafiltrationsmodus für ein vorgegebenes erstes Zeitintervall und in dem Entlüftungsmodus für ein vorgegebenes zweites Zeitintervall betrieben wird.

5. Vorrichtung zur extrakorporalen Blutbehandlung nach Anspruch 3 oder 4, **dadurch gekennzeichnet, dass** die erste Luftabscheideeinrichtung (12) einen Füllstandgeber (12E) aufweist, wobei die Steuereinheit (23) derart konfiguriert ist, dass die Ultrafiltrationseinrichtung (15) in dem Ultrafiltrationsmodus und dem Entlüftungsmodus solange wechselweise betrieben wird, bis der Füllstandgeber (12E) einen vorgegebenen Füllstand misst.

6. Vorrichtung zur extrakorporalen Blutbehandlung nach einem der Ansprüche 2 bis 5, **dadurch gekennzeichnet, dass** das Behältnis (12A) der ersten Luftabscheideeinrichtung (12) einen Entlüftungsanschluss (12F) zum Abführen von Luft und einen Ultrafiltrationsanschluss (12G) zum Zuführen oder Abführen von verbrauchter Dialysierflüssigkeit aufweist, wobei der Entlüftungsanschluss (12F) oberhalb des Ultrafiltrationsanschlusses (12G) angeordnet ist, und das Behältnis (13A) der zweiten Luftabscheideeinrichtung (13) einen Einlass (13B) und einen Auslass (13C) aufweist, wobei der Einlass (13B) unterhalb des Auslasses (13C) angeordnet ist, und einen Ultrafiltrationsanschluss (13D) zum Zuführen oder Abführen von verbrauchter Dialysierflüssigkeit aufweist, wobei der Ultrafiltrationsanschluss (13D) unterhalb des Auslasses (13C) angeordnet ist.

7. Vorrichtung zur extrakorporalen Blutbehandlung nach Anspruch 6, **dadurch gekennzeichnet, dass** die Ultrafiltrationseinrichtung (15) eine Ultrafiltrationspumpe (16) mit einem ersten und einem zweiten Anschluss (16A, 16B) aufweist, wobei der erste Anschluss (16A) über einen ersten Abschnitt (17A) einer Ultrafiltrationsleitung (17) an den Ultrafiltrationsanschluss (12G) des Behältnisses (12A) der ersten Luftabscheideeinrichtung (12) angeschlossen ist und der zweite Anschluss (16B) über einen zweiten Abschnitt (17B) der Ultrafiltrationsleitung (17) an den Ultrafiltrationsanschluss (13D) des Behältnisses (13B) der zweiten Luftabscheideeinrichtung (13) angeschlossen ist, und
der Entlüftungsanschluss (12F) des Behältnisses (12A) der ersten Luftabscheideeinrichtung (12) über eine Entlüftungsleitung (14) an den Einlass (13B) des Behältnisses (13) der zweiten Luftabscheideeinrichtung (13) angeschlossen ist und der Auslass (13C) des Behältnisses (13) der zweiten Luftabscheideeinrichtung (13) über einen dritten Abschnitt (17C) der Ultrafiltrationsleitung (17) mit dem Ablauf (2) verbunden ist, wobei in der Entlüftungsleitung (14) ein erstes Absperrorgan (19) und in dem dritten Abschnitt (17c) der Ultrafiltrationsleitung (17) ein zweites Absperrorgan (20) angeordnet ist.

8. Vorrichtung zur extrakorporalen Blutbehandlung nach Anspruch 7, **dadurch gekennzeichnet, dass** die Steuereinheit (23) derart konfiguriert ist, dass in dem Ultrafiltrationsmodus das erste Absperrorgan (19) geschlossen und das zweite Absperrorgan (20) geöffnet ist.

9. Vorrichtung zur extrakorporalen Blutbehandlung nach Anspruch 7 oder 8, **dadurch gekennzeichnet, dass** die Steuereinheit (23) derart konfiguriert ist, dass in dem Entlüftungsmodus das erste Absperrorgan (19) geöffnet und das zweite Absperrorgan (20) geschlossen ist.

## Claims

1. Apparatus for extracorporeal blood treatment, comprising
a device (1) for providing fresh dialysate and a drain (2) for used dialysate,
a balancing device (4) for balancing fresh dialysate and used dialysate,
a dialyser (3), which is divided into a blood chamber (6) and a dialysate chamber (7) by a semi-permeable membrane (5),
a dialysate supply line (10) for fresh dialysate, leading from the device (1) for providing fresh dialysate to an inlet of the dialysate chamber (7) via the balancing device (4), and a dialysate discharge line (11) for used dialysate, leading from an outlet of the dialysate chamber (7) to the drain (2) via the balancing device (4),
a first air separation device (12) comprising a container (12A) having an inlet (12B) for supplying used dialysate and an outlet (12C) for discharging used dialysate, which are arranged such that a liquid level (12D) forms in the container (12A), the dialysate discharge line (11) comprising a first line portion (11A) that leads from the outlet of the dialysate chamber (7) to the inlet (12A) of the first air separation device (12), and comprising a second line portion (11B) that leads from the outlet of the first air separation device (12) to the drain (2) via the balancing device (4),
an ultrafiltration device (15) for removing used dialysate from the container (12A) of the first air separation device (12) and for supplying the used dialysate to the drain (2) while bypassing the balancing device (4),
the ultrafiltration device (15) being designed such that, the ultrafiltration device (15) is operated in an ultrafiltration mode to remove used dialysate from the container (12A) of the first air separation device (12),
**characterised in that** in a ventilation mode the ultrafiltration device (15) is operated in the opposite direction to supply used dialysate into the container (12A) of the first air separation device (12) such that the liquid level (12D) rises in the container of the first air separation device.

2. Apparatus for extracorporeal blood treatment according to claim 1, **characterised in that** a second air separation device (13) is provided and comprises a container (13A) that is in fluid communication with the first air separation device (12) and the ultrafiltration device (15) in such a way that the air discharged from the first air separation device (12) in the ventilation mode when the fluid level (12D) rises is collected in the container (13A) of the second air separation device (13), whilst the ultrafiltration device (15) is operated in the opposite direction to supply used dialysate into the container (12A) of the first air separation device (12).

3. Apparatus for extracorporeal blood treatment according to either claim 1 or claim 2, **characterised in that** the blood treatment apparatus comprises a control unit (23) which is designed such that the ultrafiltration device (15) is operated alternately in the ultrafiltration mode and in the ventilation mode.

4. Apparatus for extracorporeal blood treatment according to claim 3, **characterised in that** the control unit (23) is designed such that the ultrafiltration device (15) is operated in the ultrafiltration mode for a predetermined first period of time and in the ventilation mode for a predetermined second period of time.

5. Apparatus for extracorporeal blood treatment according to either claim 3 or claim 4, **characterised in that** the first air separation device (12) comprises a level indicator (12E), the control unit (23) being designed such that the ultrafiltration device (15) is operated alternately in the ultrafiltration mode and the ventilation mode until the level indicator (12E) measures a predetermined level.

6. Apparatus for extracorporeal blood treatment according to any of claims 2 to 5, **characterised in that** the container (12A) of the first air separation device (12) comprises a ventilation port (12F) for discharging air and an ultrafiltration port (12G) for supplying or discharging used dialysate, the ventilation port (12F) being arranged above the ultrafiltration port (12G), and the container (13A) of the second air separation device (13) comprises an inlet (13B) and an outlet (13C), the inlet (13B) being arranged below the outlet (13C), and an ultrafiltration port (13D) for supplying or discharging used dialysate, the ultrafiltration port (13D) being arranged below the outlet (13C).

7. Apparatus for extracorporeal blood treatment according to claim 6, **characterised in that** the ultrafiltration device (15) comprises an ultrafiltration pump (16) having a first and a second port (16A, 16B), the first port (16A) being connected to the ultrafiltration port (12G) of the container (12A) of the first air separation device (12) via a first portion (17A) of an ultrafiltration line (17) and the second port (16B) being connected to the ultrafiltration port (13D) of the container (13B) of the second air separation device (13) via a second portion (17B) of the ultrafiltration line (17), and
the ventilation port (12F) of the container (12A) of the first air separation device (12) is connected to the inlet (13B) of the container (13) of the second air separation device (13) via a ventilation line (14), and the outlet (13C) of the container (13) of the second air separation device (13) being connected to the drain (2) via a third portion (17C) of the ultrafiltration line (17), a first valve (19) being arranged in the ventilation line (14) and a second valve (20) being arranged in the third portion (17C) of the ultrafiltration line (17).

8. Apparatus for extracorporeal blood treatment according to claim 7, **characterised in that** the control unit (23) is designed such that the first valve (19) is closed and the second valve is open (20) in the ultrafiltration mode.

9. Apparatus for extracorporeal blood treatment according to either claim 7 or claim 8, **characterised in that** the control unit (23) is designed such that the first valve (19) is open and the second valve (20) is closed in the ventilation mode.

## Revendications

1. Dispositif de traitement extracorporel du sang avec
un système (1) pour la fourniture de liquide de dialyse frais et une sortie (2) pour du liquide de dialyse utilisé,
un système d'équilibrage (4) pour l'équilibrage de liquide de dialyse frais et de liquide de dialyse utilisé,
un dialyseur (3) qui est divisé par une membrane semi-perméable (5) en une chambre de sang (6) et une chambre de liquide de dialyse (7),
une conduite d'amenée de liquide de dialyse (10) menant du système (1) pour la fourniture de liquide de dialyse frais par le biais du système d'équilibrage (4) à une entrée de la chambre de liquide de dialyse (7) pour du liquide de dialyse frais, et une conduite d'évacuation de liquide de dialyse (11) menant d'une sortie de la chambre de liquide de dialyse (7) par le biais du système d'équilibrage (4) à la sortie (2) pour du liquide de dialyse utilisé,
un premier système de séparation d'air (12) qui présente un récipient (12A) avec une entrée (12B) pour l'amenée de liquide de dialyse utilisé et une sortie (12C) pour l'évacuation de liquide de dialyse utilisé, qui sont disposées de telle manière qu'un niveau de liquide (12D) se forme dans le récipient (12A), dans lequel la conduite d'évacuation de liquide de dialyse (11) présente une première section de conduite (11A) qui mène de la sortie de la chambre de liquide de dialyse (7) à l'entrée (12A) du premier système de séparation d'air (12), et une seconde section de conduite (11B) qui mène de la sortie du premier système de séparation d'air (12) par le biais du système d'équilibrage (4) à la sortie (2),
un système d'ultrafiltration (15) pour le retrait de liquide de dialyse utilisé du récipient (12A) du premier système de séparation d'air (12), et l'amenée du liquide de dialyse utilisé à la sortie (2) en contournant le système d'équilibrage (4),
dans lequel
le système d'ultrafiltration (15) est réalisé de telle manière que dans un mode d'ultrafiltration le système d'ultrafiltration (15) soit actionné pour le retrait de liquide de dialyse utilisé du récipient (12A) du premier système de séparation d'air (12), **caractérisé en ce que** dans un mode d'aération le système d'ultrafiltration (15) est actionné dans le sens inverse pour l'amenée de liquide de dialyse utilisé dans le récipient (12A) du premier système de séparation d'air (12) de sorte que le niveau de liquide (12D) augmente dans le récipient du premier système de séparation d'air.

2. Dispositif de traitement extracorporel du sang selon la revendication 1, **caractérisé en ce qu'**un second système de séparation d'air (13) est prévu, lequel présente un récipient (13A) qui est en liaison fluidique avec le premier système de séparation d'air (12) et le système d'ultrafiltration (15), de telle manière que l'air évacué dans le mode d'aération lors de l'augmentation du niveau de liquide (12D) du premier système de séparation d'air (12) soit accumulé dans le récipient (13A) du second système de séparation d'air (13) alors que le système d'ultrafiltration (15) est actionné dans le sens inverse pour l'amenée de liquide de dialyse utilisé dans le récipient (12A) du premier système de séparation d'air (12).

3. Dispositif de traitement extracorporel du sang selon la revendication 1 ou 2, **caractérisé en ce que** le dispositif de traitement du sang présente une unité de commande (23) qui est configurée de telle manière que le système d'ultrafiltration (15) soit actionné alternativement dans le mode d'ultrafiltration et dans le mode d'aération.

4. Dispositif de traitement extracorporel du sang selon la revendication 3, **caractérisé en ce que** l'unité de commande (23) est configurée de telle manière que le système d'ultrafiltration (15) soit actionné dans le mode d'ultrafiltration pour un premier intervalle de temps prédéfini et dans le mode d'aération pour un second intervalle de temps prédéfini.

5. Dispositif de traitement extracorporel du sang selon la revendication 3 ou 4, **caractérisé en ce que** le premier système de séparation d'air (12) présente un indicateur de niveau (12E), dans lequel l'unité de commande (23) est configurée de telle manière que le système d'ultrafiltration (15) soit actionné dans le mode d'ultrafiltration et le mode d'aération alternativement jusqu'à ce que l'indicateur de niveau (12E) mesure un niveau prédéfini.

6. Dispositif de traitement extracorporel du sang selon l'une des revendications 2 à 5, **caractérisé en ce que** le récipient (12A) du premier système de séparation d'air (12) présente un raccord d'aération (12F) pour l'évacuation d'air et un raccord d'ultrafiltration (12G) pour l'amenée ou l'évacuation de liquide de dialyse utilisé, dans lequel le raccord d'aération (12F) est agencé au-dessus du raccord d'ultrafiltration (12G), et le récipient (13A) du second système de séparation d'air (13) présente une entrée (13B) et une sortie (13C), dans lequel l'entrée (13B) est agencée en dessous de la sortie (13C), et présente un raccord d'ultrafiltration (13D) pour l'amenée ou l'évacuation de liquide de dialyse utilisé, dans lequel le raccord d'ultrafiltration (13D) est agencé en dessous de la sortie (13C).

7. Dispositif de traitement extracorporel du sang selon la revendication 6, **caractérisé en ce que** le système d'ultrafiltration (15) présente une pompe d'ultrafiltration (16) avec un premier et un second raccord (16A, 16B), dans lequel le premier raccord (16A) est raccordé par le biais d'une première section (17A) d'une conduite d'ultrafiltration (17) au raccord d'ultrafiltration (12G) du récipient (12A) du premier système de séparation d'air (12) et le second raccord (16B) est raccordé par le biais d'une deuxième section (17B) de la conduite d'ultrafiltration (17) au raccord d'ultrafiltration (13D) du récipient (13B) du second système de séparation d'air (13), et
le raccord d'aération (12F) du récipient (12A) du premier système de séparation d'air (12) est raccordé par le biais d'une conduite d'aération (14) à l'entrée (13B) du récipient (13) du second système de séparation d'air (13) et la sortie (13C) du récipient (13) du second système de séparation d'air (13) est reliée par le biais d'une troisième section (17C) de la conduite d'ultrafiltration (17) à la sortie (2), dans lequel un premier organe de verrouillage (19) est agencé dans la conduite d'aération (14) et un second organe de verrouillage (20) est agencé dans la troisième section (17c) de la conduite d'ultrafiltration (17).

8. Dispositif de traitement extracorporel du sang selon la revendication 7, **caractérisé en ce que** l'unité de commande (23) est configurée de telle manière que dans le mode d'ultrafiltration le premier organe de verrouillage (19) est fermé et le second organe de verrouillage (20) est ouvert.

9. Dispositif de traitement extracorporel du sang selon la revendication 7 ou 8, **caractérisé en ce que** l'unité de commande (23) est configurée de telle manière que dans le mode d'aération le premier organe de verrouillage (19) est ouvert et le second organe de verrouillage (20) est fermé.
